# EUROPEAN PATENT SPECIFICATION

(11) **EP 3 140 396 B1**
(45) Date of publication and mention of the grant of the patent: **01.08.2018**
(21) Application number: 15732942.6
(22) Date of filing: 08.05.2015
(51) Int. Cl.: C12N 5/078, C12N 5/0789, A61K 41/00, A61K 33/40

(54) **METHOD FOR EXPANDING ADULT STEM CELLS FROM WHOLE BLOOD**
VERFAHREN ZUR VERMEHRUNG VON ADULTEN STAMMZELLEN AUS VOLLBLUT
PROCÉDÉ D'EXPANSION DE CELLULES SOUCHES ADULTES À PARTIR DE SANG TOTAL

(30) Priority: 09.05.2014 IT UD20140075
(43) Date of publication of application: 15.03.2017
(73) Proprietor: THANKSTEM S.R.L., 33100 Udine (UD) (IT)
(72) Inventor: POLETTINI, Marco, 01015 Viterbo (IT)
(74) Representative: Petraz, Gilberto Luigi
(86) International application number: PCT/IB2015/053379
(87) International publication number: WO 2015/170291

(56) References cited:
- WO-A1-2008/034740
- WO-A1-2009/115522
- WO-A2-2008/036374
- WO-A2-2009/046377
- SPAAS J.H., GAMBACURTA A., POLETTINI M., BROECKX S., ET AL.: "Purification and expansion of stem cells from equine peripheral blood, with clinical applications.", , 2011, pages 129-135, XP002728029, Retrieved from the Internet: URL:http://hdl.handle.net/1854/LU-1215157 [retrieved on 2014-07-29]
- G. E. Garber ET AL: "The use of ozone-treated blood in the therapy of HIV infection and immune disease: a pilot study of safety and efficacy.", AIDS, 1 January 1991 (1991-01-01), pages 981-984, XP055132379, Retrieved from the Internet: URL:http://graphics.tx.ovid.com/ovftpdfs/F PDDNCFBHADJCP00/fs047/ovft/live/gv039/0000 2030/00002030-199108000-00009.pdf [retrieved on 2014-07-30]
- LARINI A ET AL: "Effects of ozone on isolated peripheral blood mononuclear cells", TOXICOLOGY IN VITRO, ELSEVIER SCIENCE, GB, vol. 19, no. 1, 1 February 2005 (2005-02-01), pages 55-61, XP027831744, ISSN: 0887-2333 [retrieved on 2005-02-01]

## Description

### FIELD OF THE INVENTION

Forms of embodiment described here concern a method for expanding adult stem cells from whole blood, in particular but not only, peripheral blood, from adult mammals, and the corresponding application in the medical field, in particular in human or veterinary medicine, for the therapeutic treatment of lesions, both external and internal, lesions to tendons, to ligaments and to cartilage, bone fractures, and also the therapeutic and/or preventive treatment of chronic and/or acute inflammatory pathologies, neurological and neurodegenerative pathologies, cardiac pathologies, tumorous pathologies, autoimmune pathologies, ophthalmic pathologies and pathologies of a genetic origin.

Here and hereafter in the description, and as known in literature, the word "expansion" means the process to increase the number of cells, either by cell division or, as in the specific case described and claimed here, by "de-differentiation" or "de-programming", that is to say, the process by which some cells present in the blood are re-transformed into stem cells following suitable in vitro treatment, as will be seen hereafter.

### BACKGROUND OF THE INVENTION

In recent years the use of stem cells in therapy has received widespread consensus, but the therapeutic results obtained are far below expectations, except for stem cells obtained from blood.

In fact, many known methods for obtaining stem cells have proven to be long, laborious and expensive, with relative results and sometimes collateral effects.

There are embryonic stem cells and adult stem cells: the former derive from 8-day blastocysts, while the adult ones can be obtained mainly from bone marrow, adipose or muscular tissue, from peripheral blood and from the umbilical cord, etc...

The definition of stem cells is constantly evolving. For all these cells, both embryonic (ES) and adult, both hematopoietic (HSC) and mesenchymal (MSC) (Kuwana M. et al., 2003), various genetic markers have been identified, of which some are common to many cell types (Condomines M. et al., 2006; Kang W. J. et al., 2006; Zhao Y. et al., 2003; Rabinovitch M. et al., 1976).

To identify pluripotent stem cells (PSCs), embryonic and adult, the expression of some intracellular transcription factors is considered (Sox2,Oct3/4 and Nanog).

Initially, research was directed toward stem cells of embryonic origin, because they are pluripotent, and also qualifiable and quantifiable, thus suitable for an experimental trial; however, ethical questions, and above all the counter-indications due to the production of tumors, meant they had to be set aside. Therefore, nowadays, adult stem cells are preferred. Adult stem cells of another individual (allogenic) very frequently cause serious problems of rejection, because they are not recognized as "self'. This especially affects stem cells from the umbilical cord, which are used almost exclusively as allogenic stem cells.

Pluripotent stem cells induced using a process that transfers through viruses the pluripotence factors from embryonic stem cells to adult stem cells are cells that are not suitable for treatment due to the counter-indications similar to those had with embryonic stem cells, and to the extremely high costs.

In man, for now, the use of stem cells obtained from peripheral blood through a process called "apheresis" or "leukapheresis" is accepted. The stem cells are extracted from the blood, collected, and then inoculated into patients affected by some leukemic pathologies, immediately after chemotherapy or radiotherapy. The stem cells are hematopoietic, and so they inter-react exclusively with pathologies of the blood.

In apheresis, which lasts from 6 to 8 hours, the blood is taken from a vein in the arm, neck or chest, and made to pass through a machine that removes the stem cells. The blood, thus purified, returns to the patient, while the collected cells are preserved through refrigeration in liquid nitrogen (Condomines M. et al., 2006; Kang W.J. et al., 2006). This technique is not only painful, but is also extremely stressful for the patient. It provides an *in vivo* inoculation of growth factors to stimulate the release of stem cells from the bone marrow to the blood, and does not allow a real discrimination and/or purification of the stem cells in circulation.

These cells are allowed, nowadays, according to legislation, in therapies for treating pathologies exclusively of the blood.

The stem cells that are being introduced onto the market today to treat different pathologies are adult stem cells, mainly mesenchymal, obtained from bone marrow and from fat. However, these have certain limits:
- they require invasive methods to collect them, drilling a bone for bone marrow stem cells or surgery for stem cells from fat;
- they are able to inter-react only with some tissues due to their mesenchymal derivation;
- when they are cultivated to obtain an adequate number for therapy, they begin to differentiate into other cell types, showing always different membrane receptors and they cannot therefore be qualified and quantified, which are indispensable characteristics for a human trial.

Another known method is described by Zhao Y. et al., in the article "A human peripheral blood monocyte-derived subset acts as pluripotent stem cells" and in WO-A-2004/043990. This is a method for preparing stem cells deriving from monocytes, which includes the steps of isolating monocytes from peripheral blood, putting them into contact with a mitogenic component and subsequently cultivating the monocytes from peripheral blood in conditions suitable to propagate the cells.

This method, which initially requires a step of isolating the monocyte and then an expansion step in a culture medium, is very long, about 15 - 20 days, to obtain a significant number of stem cells, and does not allow to obtain pluripotent stem cells.

Again in the framework of preparing stem cells from monocytes, documents WO-A-2005/046570, WO-A-2007/131200 and WO-A-03/083092 are known. However, since they have to perform a preliminary purification of the blood in order to isolate only one cell fraction, that is, the monocytes, and a subsequent expansion to obtain the desired stem cells, the methods described in these documents also require a very long time, again in the order of 15 - 40 days, to obtain an acceptable quantity of stem cells.

Document WO-A-2008/034370 in the name of the present Applicant is also known, and concerns an expansion method for adult stem cells from blood, using a Macrophage Colony Stimulating Factor (MCSF). This known method provides to grow the adult blood stem cells after the blood has been taken by an in vitro treatment with MCSF, in a concentration comprised between 8 nM and 15 nM, and a subsequent purification, preferably by fractioning on a Ficoll gradient. The method can also provide to grow the stem cells from peripheral blood purified by the in vitro treatment with MCSF in a concentration comprised between 35 nM and 55 nM.

The efficacy of the method is confirmed by the presence and recognition of stem cell markers CD90, CD90/34, CD34 and CD 117, and by the fact that the stem cells do not lose their "self' recognition factors following division or expansion. The stem cells do not give rise to collateral effects such as rejection, infection or development of teratomas when they have been administered to the patient, and are able to differentiate "in vivo" and behave like pluripotent stem cells.

The authors have seen that cells thus grown, through division or expansion, when injected locally or intravenously, acquire "in vivo" (and not "in vitro" as in known methods in the state of the art by suitable growth factors and/or chemical stimuli (Gulati R. et al., 2003; Katz R. L. et al., 2002; Okazaki T. et al, 2005)), all the morphological and chemical characteristics of macrophage, lymphocyte, epithelium, endothelium, neuronal and hepatocyte cells, depending on the needs and pathologies of the living organisms treated. The method is less invasive than other methods used before to collect stem cells, it is painless (unlike apheresis) and economical.

Finally, the possibility of obtaining these cells easily, and then being able to preserve them for a long time, for example frozen in liquid nitrogen, makes the cells obtained using this known method suitable for autologous transplants and in the treatment of many pathologies (lesions of various types, metabolic illnesses, neurological pathologies, acute and chronic inflammatory pathologies).

Document WO-A-2009/115522 is also known, in the name of the present Applicant, and concerns a kit for collecting blood, preferably peripheral blood, for the production of pluripotent stem cells, including a container, able to contain the blood taken, which contains an anti-coagulant and the MCSF substance. The kit can be used in the framework of the method described in WO-A-2008/034370.

However, Applicant has found that the various working and handling steps to which the stem cells are subjected in the execution of the method described in WO-A-2008/034370, such as the elimination of the red corpuscles, the purification of the stem cells with respect to the other components of the blood, obtaining a greater quantity of pluripotent stem cells compared with hematopoietic and mesenchymal stem cells, cultivation, differentiation into other cell types, can stress the adult stem cells thus obtained, leaving them alive but less efficacious and with a reduced potential capacity for energy and information.

In the veterinary field there are various techniques and apparatuses for producing stem cells, in particular to concentrate stem cells from fat and bone marrow and to obtain growth factors.

However, for stem cells a first obstacle is that they are difficult to collect: as we said, to obtain them from bone marrow a bone must be drilled or the backbone penetrated, and to obtain them from fat a surgical operation proper is required, with stitches.

There is also the logistical complexity: dispatching the sample and receiving the stem cells, keeping them all alive and stable.

Other obstacles can be the number of manual operations required, the preparation time and costs for all the adult stem cells used until now.

These difficulties, together with poor clinical results, have caused stem cells to almost completely disappear from veterinary clinical practice, and only a few veterinarians continue to use them, above all for experimental purposes.

The cell preparation described in WO-A-2008/034370 and reported in various scientific publications is able to qualify and quantify the stem cells obtained, confirming their pluripotent characteristics. It is also much easier than the techniques used until now, both because sampling is simple - a few milliliters of blood - and also because the cells do not have to be cultivated.

However, this system too can be improved, to use stem cells in human clinical practice, to overcome obstacles connected to the dispatch of the sample to the lab, the subsequent de-programming with MCSF and the return to the structures where the therapeutic treatment is performed. Another limit can be the complete purification of the stem cells obtained from blood using the method described in WO-A-2008/034370 which allows greater safety for a possible allogenic inoculation (the safety of which is still to be proven). In fact, purifications and processes used to eliminate the red corpuscles and the passage of the cells in a sorter (qualification) create considerable stress for the stem cells obtained, making them lose part of their curative capacity. Therefore, there is a strong need to reduce to a minimum the handling of the blood to obtain effective stem cells and thus obtain better results.

Another limit of all the therapies with stem cells obtained using known methods, including WO-A-2008/034370, is that they require specialized laboratories.

Document WO-A-2008/036374 is also known, which describes methods and compositions for transplants of stem cells in patients who have not been previously immune-suppressed.

The following scientific articles are also known:
- Spaas J. H., Gambacurta A., Polettini M., Broeckx S. et al., "Purification and expansion of stem cells from equine peripheral blood, with clinical applications", vol. 80, no. 2, pages 129-135 retrieved from the Internet: URL:http://hdl.handle.net/1854/LU-1215157;
- G. E. Garber et al., "The use of ozone-treated blood in the therapy of HIV infection and immune disease: a pilot study of safety and efficacy" AIDS, 1 January 1991, pages 981-984, retrieved from the Internet: URL:http://graphics.tx.ovid.com/ovftpdfs/FPDDNCFBHADJCP00/fs047/ovft/liv e/gv039/00002030/00002030-199108000-00009.pdf;
- Larini et al., "Effects of ozone on isolated peripheral blood mononuclear cells", Toxicology in vitro, Elsevier Science, GB, vol. 19, no. 1, 1 February 2005, pages 55-61.

There is therefore a need to perfect a method for expanding adult stem cells from whole blood that can overcome at least one of the disadvantages of the state of the art.

The Applicant has devised, tested and embodied the present invention to overcome the shortcomings of the state of the art and to obtain these and other purposes and advantages.

Unless otherwise defined, all the technical and scientific terms used here and hereafter have the same meaning as commonly understood by a person with ordinary experience in the field of the art to which the present invention belongs. Even if methods and materials similar or equivalent to those described here can be used in practice and in the trials of the present invention, the methods and materials are described hereafter as an example. In the event of conflict, the present application shall prevail, including its definitions. The materials, methods and examples have a purely illustrative purpose and shall not be understood restrictively.

### SUMMARY OF THE INVENTION

The present invention is set forth and characterized in the independent claims, while the dependent claims describe other characteristics of the invention or variants to the main inventive idea.

In accordance with the above purposes, a method for expanding adult stem cells from blood, which overcomes the limits of the state of the art and eliminates the defects therein, provides:
- growth and de-programming of the adult blood stem cells of a blood sample which has been taken, using in vitro treatment of the blood sample with Macrophage Colony Stimulating Factor (MCSF);
- ozonization of the blood sample.

The present invention thus allows to obtain pluripotent adult stem cells from the blood sample.

According to possible forms of embodiment, the method provides that the ozonization of the blood sample is carried out before the MCSF treatment. In particular, the treatment with MCSF can be made on the already ozonized blood sample.

According to other possible forms of embodiment, the method provides that the ozonization of the blood sample is carried out during the MCSF treatment. In particular, the treatment with MCSF can be made on the blood sample during ozonization.

According to other possible forms of embodiment, the method provides that the ozonization of the blood sample is carried out after the MCSF treatment. In particular, the treatment with MCSF can be made on the blood sample before it is ozonized.

According to possible forms of embodiment, which can be combined with all the forms of embodiment described here, the method provides that the ozonization supplies to the blood sample a mixture of O₂ - O₃.

According to possible forms of embodiment, which can be combined with all the forms of embodiment described here, the method provides a stoichiometric ratio of blood to the O₂ - O₃ mixture of 1:1.

According to possible forms of embodiment, which can be combined with all the forms of embodiment described here, the method provides a quantity of O₂ - O₃ mixture in the blood sample greater than or equal to about 1 mic.g/l.

According to possible forms of embodiment, which can be combined with all the forms of embodiment described here, the method provides that the quantity of O₂ - O₃ mixture in the blood sample can be selected in an interval from about 1 mic.g/ml to about 42 mic.g/ml.

According to possible forms of embodiment, which can be combined with all the forms of embodiment described here, the method provides to add an anti-coagulant to the blood sample.

According to possible forms of embodiment, which can be combined with all the forms of embodiment described here, the method provides to use a kit to collect blood which includes at least a container able to contain at least the blood taken, containing at least the MCSF substance.

According to possible forms of embodiment, which can be combined with all the forms of embodiment described here, the method provides that the quantity of blood sample collected and treated is comprised between 0.2 ml and 100 ml, in particular between 0.5 ml and 50 ml, more particularly between 1 ml and 25 ml, still more particularly between 2 ml and 10 ml, more particularly between 2 ml and 8 ml, more particularly between 3 ml and 8 ml, more particularly between 3 ml and 5 ml.

According to possible forms of embodiment, which can be combined with all the forms of embodiment described here, the method provides that the concentration of MCSF is comprised in an interval from about 1 nM to about 55 nM.

According to possible forms of embodiment, which can be combined with all the forms of embodiment described here, the method provides a growth and de-programming time by means of in vitro treatment with MCSF comprised between 4 hours and 96 hours.

Furthermore, other forms of embodiment described here concern a method for expanding adult stem cells from blood, which consists exclusively of:
- growth and de-programming of the adult blood stem cells of a blood sample which has been taken, using in vitro treatment of the blood sample with MCSF.

Forms of embodiment described here also concern a blood sample containing adult stem cells obtainable by means of a method according to the present description.

According to possible forms of embodiment, the blood sample is provided for use in the therapeutic treatment and/or prevention of pathologies.

According to possible forms of embodiment, the blood sample is provided for use in therapeutic treatment including the therapy of lesions, both external and internal, lesions of the tendons, of the ligaments and of the cartilages, bone fractures, the therapy and/or prevention of chronic and/or acute inflammatory pathologies, neurological and neurodegenerative pathologies, cardiac pathologies, tumorous pathologies, autoimmune pathologies, ophthalmic pathologies and genetic pathologies.

According to possible forms of embodiment, a blood sample is provided for use in a treatment that provides intravenous, intra-arterial or local administration (for example subcutis, intramuscular or intra-tissue) of the blood sample treated with MCSF and ozonized.

According to other possible forms of embodiment, a blood sample is provided for use in a treatment that provides intravenous or intra-arterial or local administration (for example subcutis, intramuscular or intra-tissue) of the blood sample treated with MCSF and the systemic ozonization of the patient.

Forms of embodiment described here also concern a kit including at least a container containing a blood sample containing adult stem cells obtainable using a method according to the present description.

These and other aspects, characteristics and advantages of the present disclosure will be better understood with reference to the following description, drawings and attached claims. The drawings, which are integrated and form part of the present description, show some forms of embodiment of the present invention, and together with the description, are intended to describe the principles of the disclosure.

The various aspects and characteristics described in the present description can be applied individually where possible. These individual aspects, for example aspects and characteristics described in the attached dependent claims, can be the object of divisional applications.

It is understood that any aspect or characteristic that is discovered, during the patenting process, to be already known, shall not be claimed and shall be the object of a disclaimer.

### DETAILED DESCRIPTION OF SOME FORMS OF EMBODIMENT

We shall now refer in detail to the various forms of embodiment of the present invention. Each example is supplied by way of illustration of the invention and shall not be understood as a limitation thereof. For example, the characteristics shown or described insomuch as they are part of one form of embodiment can be adopted on, or in association with, other forms of embodiment to produce another form of embodiment. It is understood that the present invention shall include all such modifications and variants.

Before describing these forms of embodiment, we must also clarify that the present description is not limited in its application to details of the construction and disposition of the components as described in the following description using the attached drawings. The present description can provide other forms of embodiment and can be obtained or executed in various other ways. We must also clarify that the phraseology and terminology used here is for the purposes of description only, and cannot be considered as limitative.

Terms such as "about", "generally", "substantially" and suchlike shall be understood with their function of modifying a term or value that is not absolute, but is not reported in the state of the art. Such terms shall be defined by the specific circumstances and by the terms that they are intended to modify according to the common acceptance of such terms in the specific field. They shall take into account at least the degree of experimental error expected, the technical error and the instrumental error for a given technique adopted to measure a value. Unless otherwise indicated, in the present description, singular forms such as "a", "an" and "one" shall be understood to include plural forms, unless the context suggests otherwise.

All the intervals reported here shall be understood to include the extremes, including those that report an interval "between" two values, unless otherwise indicated.

The present description also includes the intervals that derive from uniting or overlapping two or more intervals described, unless otherwise indicated.

The present description also includes the intervals that can derive from the combination of two or more values taken at different points, unless otherwise indicated.

Unless otherwise defined, all the technical and scientific terms used here and hereafter have the same meaning as commonly understood by a person with ordinary experience in the field of the art to which the present invention belongs.

Even if methods and materials similar or equivalent to those described here can be used in practice and in the trials of the present invention, the methods and materials are described hereafter as an example. In the event of conflict, the present application shall prevail, including its definitions. The materials, methods and examples have a purely illustrative purpose and shall not be understood restrictively.

Forms of embodiment described here concern a method for expanding adult stem cells from blood, which provides:
- growth and de-programming of the adult blood stem cells of a blood sample which has been taken from the patient, using in vitro treatment of the blood sample with MCSF;
- ozonization of the blood sample.

In particular, the blood can be whole blood, more particularly peripheral whole blood.

The present invention therefore allows to obtain pluripotent adult stem cells from the blood sample taken.

In fact, as described in WO-A-2008/034370 and WO-A-2009/115522, the stem cells obtained have the stem markers CD90, CD90/34, CD34 and CD117, they also express some intra-cellular transcription factors that are strongly linked to pluripotent characteristics (Sox2,Oct3/4 and Nanog) and do not lose their "self' recognition factors following division or expansion. The stem cells do not cause collateral effects such as rejection, infection, development of teratomas once administered to the patient, they are able to differentiate themselves "in vivo" and therefore to behave like pluripotent stem cells.

The expression "growth and de-programming of the adult blood stem cells of a blood sample which has been taken from the patient, using in vitro treatment of the blood sample with MCSF" means that the blood sample which has been taken from the patient and that contains a certain quantity of adult stem cells, is treated in vitro with MCSF to obtain the growth of the adult stem cells originally present in the blood sample, by de-programming cells of the white line of the blood.

Furthermore, we underline that the expression "ozonization" here means the treatment of the blood sample with ozone, that is, the addition, delivery, administration or mixing of ozone, or a mixture of oxygen and ozone, to/in the blood sample.

Ozone (symbol O₃) is an allotropic form of oxygen, with a triatomic molecule and a molecular weight of 48. Under normal conditions ozone appears as a blue gas, with an acrid odor, and has a strong oxidizing power. Ozone can act as a disinfectant, deodorant, bactericide, sterilizer or oxidant in numerous organic syntheses.

According to possible forms of embodiment, the ozonization of the blood sample can be carried out before the MCSF treatment.

According to possible forms of embodiment, the ozonization of the blood sample can be carried out simultaneously with the MCSF treatment.

According to possible forms of embodiment, the ozonization of the blood sample can be carried out after the MCSF treatment.

According to possible forms of embodiment, it is provided to add an anti-coagulant to the blood sample. Heparin, EDTA or sodium citrate are examples of possible anti-coagulants.

In some forms of embodiment, the method according to the present description can provide to use a kit to collect the blood, for the production of pluripotent stem cells according to the method described above, including at least a container, like a test tube, able to contain the blood sample taken, containing the MCSF substance and possibly, if provided, the anti-coagulant cited.

With a kit of this type it is possible to collect whole blood, preferably peripheral blood, in order to start the growth and production of the stem cells quickly, using the method described above according to the present description and therefore to make production much quicker.

Forms of embodiment described here can provide that the quantity of blood sample collected and treated according to the method described here, that is, growth and de-programming with MCSF and ozonization of the blood sample, is just a few milliliters, for example comprised between 0.2 ml and 100 ml, in particular between 0.5 ml and 50 ml, more particularly between 1 ml and 25 ml, still more particularly between 2 ml and 10 ml, more particularly between 2 ml and 8 ml, more particularly between 3 ml and 8 ml, still more particularly between 3 ml and 5 ml.

Other variants can provide that the quantity of blood sample collected and subjected to growth and de-programming using MCSF is a few hundred milliliters, for example from 100 to 1000 ml, in particular from 200 ml to 600 ml, more particularly from 400 ml to 600 ml, for example 500 ml. The blood sample can be injected to circulate in the patient (intravenously or intra-arterially), who can subsequently be subjected to a systemic ozonization treatment.

Some forms of embodiment, which can be combined with all the forms of embodiment described here, can provide a method as described above which can use any type of container into which the whole blood and the ozone can be introduced, with any type of possible anti-coagulant and with any concentration of MCSF, for example in an interval from about 1 nM to about 55 nM. Examples of sub-intervals can be from 2 nM to 50 nM, or from 5 nM to 45 nM. Other examples of sub-intervals can be from 2 nM to 20 nM, or from 8 nM to 15 nM, or from 8 to 10 nM, or from 10 nM to 12 nM, or from 12 nM to 35 nM, or from 15 nM to 30 nM, or from 20 nM to 25 nM or from 35 nM to 55 nM or from 40 nM to 50 nM, or combinations of all these intervals or sub-intervals, also including all whole numbers or fractions present in the intervals or sub-intervals mentioned and not explicitly indicated here.

Some forms of embodiment, which can be combined with all the forms of embodiment described here, can provide that ozonization supplies the blood sample with a mixture of O₂ - O₃.

In possible implementations, Applicant has found that the ratio of blood to O₂ - O₃ mixture can preferably be a stoichiometric ratio of 1:1.

In possible implementations, the quantity of O₂ - O₃ mixture in the blood sample can be greater than or equal to about 1 mic.g/l, in particular selected in an interval from about 1 mic.g/ml to about 42 mic.g/ml, more particularly from about 5 mic.g/ml to about 30 mic.g/ml, still more particularly from about 10 mic.g/ml to about 20 mic.g/ml.

Some forms of embodiment described here provide that, having left the blood in these conditions, preferably at room temperature, after a certain time, preferably between 4 hours and 96 hours, in particular between 4 hours and 72 hours, more particularly between 4 hours and 48 hours, the whole blood thus obtained with the component of stem cells obtained from de-programming can be totally re-inoculated systemically (intravenously or intra-arterially) or locally into or near a diseased tissue.

In possible implementations, the growth and de-programming time using in vitro treatment with MCSF can be comprised between 12 hours and 96 hours, in particular between 12 hours and 72 hours, more particularly between 12 hours and 36 hours.

In possible implementations, the growth and de-programming time using in vitro treatment with MCSF can be comprised between 24 hours and 96 hours, in particular between 24 hours and 72 hours, more particularly between 24 hours and 36 hours.

In possible implementations, the growth and de-programming time using in vitro treatment with MCSF can be comprised between 48 hours and 96 hours, in particular between 48 hours and 72 hours, more particularly between 48 hours and 60 hours.

Applicant has hypothesized that ozonization of the blood sample subjected to growth and de-programming using in vitro treatment with MCSF stimulates the process of expansion and de-programming of adult stem cells, so that after a few hours there is a significant number of useful adult stem cells.

Applicant has found that a duration of the in vitro treatment with MCSF comprised between about 4 and 96 hours can lead to a stabilization of the growth of the stem cells, with identification of the stem markers CD90, CD90/34, CD34 and CD117. This is believed to be the optimum condition.

Applicant has also found that with concentrations of MCSF from about 1 nM to about 55 nM the cells maintain the phenotype of pluripotent adult stem cells. It has been observed that using MCSF in concentrations greater than 55 nM (for example 70nM), already after 24 hours the cells no longer maintain the phenotype of pluripotent adult stem cells.

Forms of embodiment of the method described here can provide not only the cited container in which there is the MCSF and where the expansion of the adult stem cells occurs, but also the use of a second container to contain the stem cells obtained as described above, for example in the case of intravenous or intra-arterial use, and possibly a third container, of different sizes, for local use. The stem cells produced and preserved in said containers can be used immediately, or can be preserved, for example in liquid nitrogen, to be used later, as needed.

Ozonization according to the present description can be carried out on blood samples containing the stem cells before, during or after expansion and de-programming with MCSF, contained in any one of the containers cited.

According to possible forms of embodiment, the blood just taken from the patient can be inserted immediately into the test tube with the anti-coagulant and MCSF. The anti-coagulant can stop the onset of coagulation, while the simultaneous presence of MCSF can allow to quickly start the expansion process and guarantee to minimize the starting times of treating the patient. Furthermore, the sample is subjected to ozonization according to the present description.

According to other possible forms of embodiment, anti-coagulant can be added to the blood taken from the patient in order to stop the coagulation of the blood that is subjected to a preservation process which does not alter its ability to produce stem cells. When necessary, the blood is taken from the place where it is preserved and is subjected to the expansion procedure of the stem cells as described above, that is, adding the MCSF substance to it, quickly obtaining the necessary quantity of stem cells. Furthermore, in this case too the sample is subjected to ozonization according to the present description.

The method according to the present description allows to overcome the disadvantages of the state of the art and entails numerous advantages.

For example, the present invention allows to prepare and handle whole blood, extremely simplifying the therapy, obviating the need for any type of cell handling done in the laboratory. In fact, the present invention excludes the necessity or possibility of making treatments for example to eliminate the red corpuscles, or to purify the stem cells with respect to all the other components of the blood, to obtain a greater quantity of pluripotent stem cells compared with the other two components of stem cells, hematopoietic and mesenchymal, or to cultivate or differentiate them into other cell types. These additional treatments can normally stress the stem cells obtained, leaving them alive but with reduced information and energy potential. On the contrary, the present invention obviates the need for all further working and handling of the blood sample, so that the adult stem cells present in the whole blood maintain their characteristics better because they are not stressed and because in the blood they can benefit from the presence of other elements that assist the regenerative process.

Applicant has found that incurable pathologies such as the degeneration of the myocardium already treated with stem cells obtained from blood through de-programming with good results as described in WO-A-2008/034370 have had a decisively more positive evolution with de-programmed stem cells in whole blood, without the additional operations and handling as described above, thus using a method that can also consist exclusively of growth and de-programming with MCSF, without additional working steps including purification, subsequent expansion, or which can include growth and de-programming with MCSF and ozonization.

The preparation of stem cells according to the method described here obviates the need for complex laboratory preparation, allowing any hospital, clinic or doctor to prepare stem cells using a simple test tube with a preferably minimum quantity of MCSF. In other words, using a single test tube into which a few ml of blood sample with MCSF are put, it is possible to treat and improve even serious pathologies such as for example the after-effects of a heart attack, or Parkinson's disease. Therefore, these results support the fact that, according to possible forms of embodiment, a method for expanding adult stem cells from blood can also consist exclusively of growing and de-programming the adult blood stem cells of a blood sample, using in vitro treatment of the blood sample with MCSF.

Furthermore, in some forms of embodiment, the addition or contribution of ozone to the blood sample, deriving from the ozonization of the blood sample, can have a catalyzing effect on the de-programming of the adult stem cells and on the quality of the stem cells obtained and their information and energy content, such that it positively influences the cell regeneration of the damaged tissues, and also gives further assurance that the product is sterile. In fact, as we said, ozone can function as a disinfectant or bactericide.

### EXPERIMENTAL CASES

Applicant conceived the idea of treating with MCSF and ozonization, that is, the addition of ozone or mixture of oxygen and ozone to expansion and "de-programming" with MCSF, following some experiments in vivo that prove the catalyzing action of the ozone in the therapy done with the de-programmed stem cells obtained from the growth step according to the present description.

### SYSTEMIC OZONIZATION

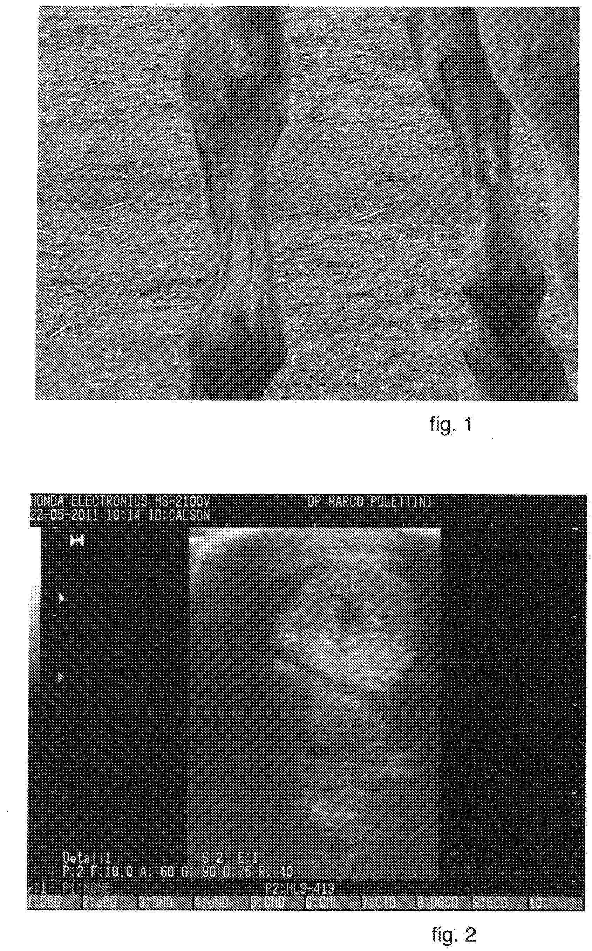

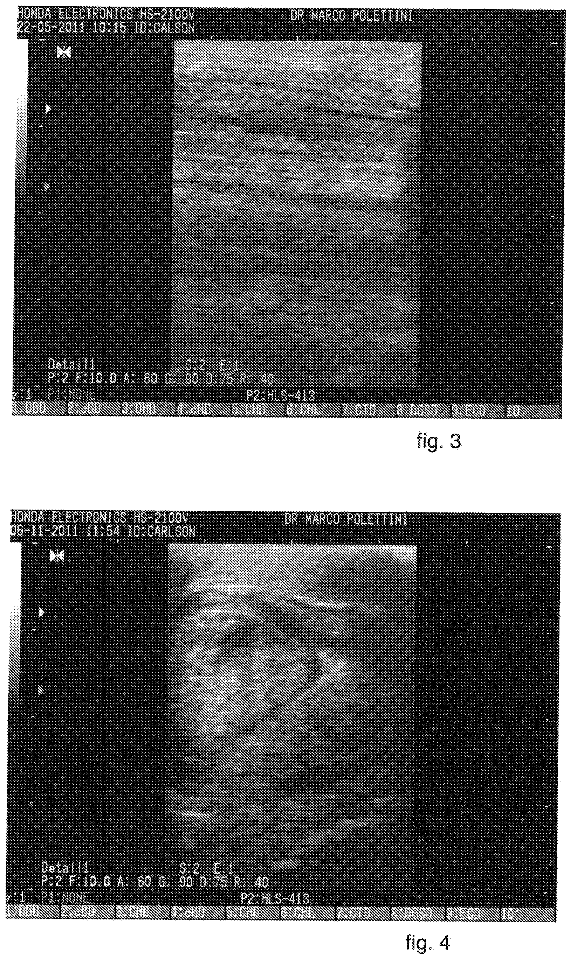

A 15-year-old horse was withdrawn from competition due to a chronic proximal lesion in the front right surface flexor tendon, which had been causing it to limp for 18 months (see fig. 1). It had been treated with "burning" 6 months before, as was common practice years ago (see fig. 2); this had not given any positive therapeutic effect and indeed had caused cicatricial sclerosis. The horse was in poor general condition and the burning had caused a proximal lesion (see fig. 3) in a sensitive area that is difficult to cure in older horses. The burning had caused a scar that not even the local injection of expanded stem cells obtained from de-programming with MCSF every 6 weeks and for three times gave any improvement, that is, not even a minimal improvement was made either in the ultrasound or in the lameness. After 5 months of such injections of expanded stem cells, the horse was made to work but the lesion had started to worsen (see figs. 4 and 5).

Therefore, in this case of tendon sclerosis due to burning, the local and systemic inoculation of de-programmed stem cells from blood obtained using expansion with MCSF, gave no benefit after three inoculations made with the indicated frequency.

Fifteen days after the third inoculation, a systemic treatment was made with ozone through an auto transfusion with half a liter of blood enriched with 120 cc of O₂ - O₃, 10 mic.g/ml.

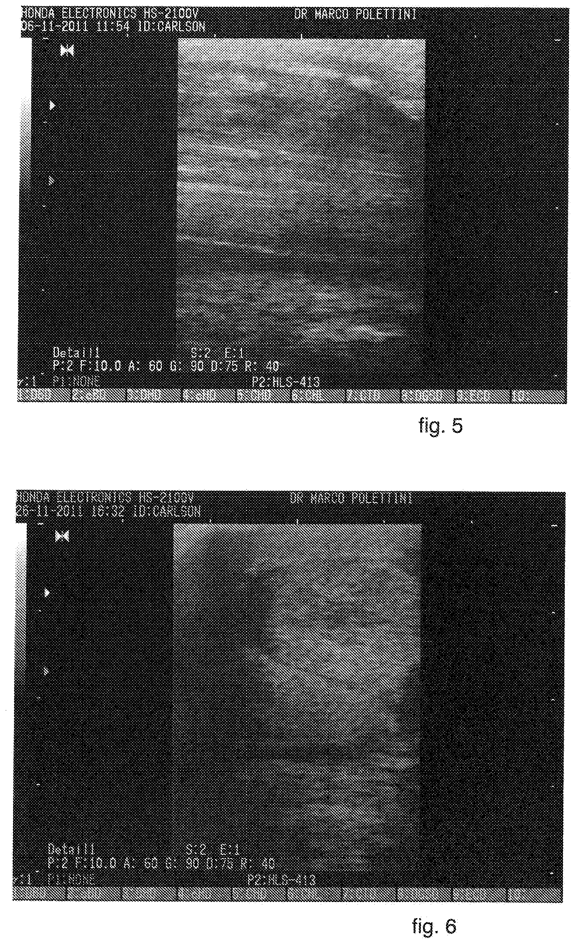

Surprisingly, Applicant found in experiments that, by introducing the ozone systemically, that is, through blood transfusion with autologous blood enriched with ozone to oxygenate the patient's blood, in only ten/fifteen days it was possible to appreciate the catalytic effect of the ozone because ultrasound showed that the lesion was resolved and the horse was no longer lame (see fig. 6). The pathological tissue, which had previously been informed by the inoculation of stem cells obtained from blood with expansion through MCSF activating the stem cells of the member of the injured surface flexor tendon, was cured by the catalyzing effect of the ozone on the regenerative process.

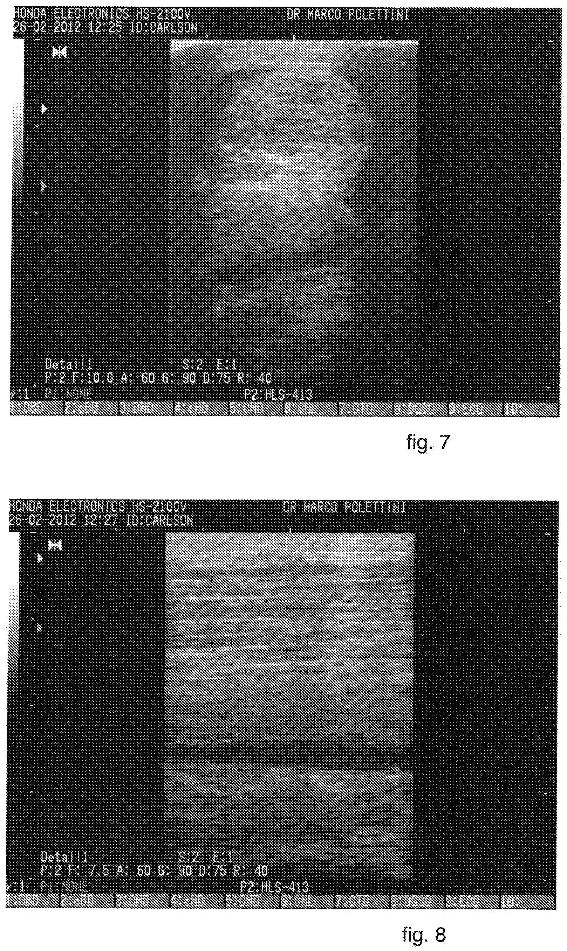

Three months after the ozone therapy, the echo-cardiograph analysis clearly showed the lesion was cured (see figs. 7 and 8).

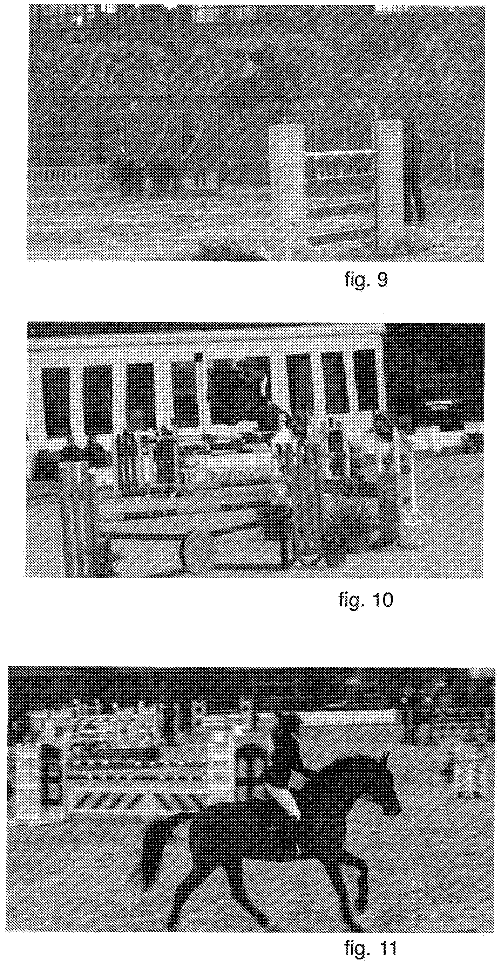

As proof of actual cure, the horse was made to work for 15 days and was sent out to compete, with positive and long-lasting effects. The improvement shown by ultrasound was real, as the horse continued regularly, with an average of 6 competitions a month, its competitive career with jumps up to one meter sixty, until the age of 18. In fact, the horse continued competing for three more years without any relapses, participating in jumping events at 16 (see fig. 9), at 17 (see fig. 10), and up to 18 years of age (see fig. 11).

The catalyzing effect of ozone on the stem cells obtained from adult blood through expansion and de-programming with MCSF was detected in vivo through the experiment described above.

After this case, ozone therapy was introduced as a catalytic agent in many patients treated with stem cells obtained from blood, expanded and de-programmed using MCSF.

The in vivo effect on stem cells obtained from blood allows to hypothesize the same catalytic effect in vitro as well, that is, in blood in a test tube, before, after or during treatment with MCSF.

### IN VITRO OZONIZATION

Following the results obtained, Applicant then also conceived the new and innovative idea of introducing the ozone directly into the container containing the blood and MCSF, so as to catalyze the de-programming process and give greater energy-information potential to the stem cells obtained by growth and de-programming with MCSF.

The effect of ozone on blood was studied by Applicant both in vitro and in vivo (see above).

When it is made to bubble in the blood, the mixture of O₂ - O₃ reacts in a few seconds with the fatty acids of the phospholipid layer of the cell membrane.

As a result of this reaction of ozone O₃ with the double link of the unsaturated fatty acids, the phospholipid chains are broken and penetrate inside the erythrocyte in the form of peroxides, influencing the reactions inside the erythrocyte, but without going beyond the cell membrane. But, due to the high cytotoxic power of peroxides, the erythrocyte reacts immediately, activating the detoxification mechanism through the glutathione system. The glutathione thus consumed is reconstructed through the glycolysis by-pass, that is, the pentose phosphate pathway.

Hence the hemoglobin (Hb) is protected from oxidation into meta-hemoglobin, keeping the function of HbO₂, allowing the transmission of oxygen O₂.

The special role played by 2,3-disphosphoglycerate (2,3-DPG) must also be considered, regarding the function of the erythrocyte. 2,3-DPG is present in erythrocytes and its function is to modulate the affinity of hemoglobin to oxygen. With ozone, in particular, the oxygen release effect is created by the erythrocyte at peripheral districts.

Another interesting action is the immuno-stimulant effect of ozone caused by the induction of interferon. Among immunocompetent cells, T4 lymphocytes or helper cells have a key role because, activated by the macrophages, they produce specific substances, interleukins, growth factors, etc., which act as intercell messengers and facilitate communication among cells.

After being activated by the interleukins, the macrophages produce the Tumor Necrosis Factor (TNF) which serves as a standard for measuring the activity of the immunocompetent cells.

Therefore, the ozone acts directly on the cells of the white line and indirectly through the reaction produced on the erythrocytes, performing a role as a catalyzer on the function of the cell lines of the blood and hence also on the de-programming process activated by the MCSF on the cells of the white line.

With regard to the ratio of blood to O₂ - O₃ mixture, Applicant found that this value is preferably a stoichiometric ratio of 1:1.

Furthermore, Applicant found that the quantity of the O₂ - O₃ mixture in the blood sample can be greater than or equal to about 1 mic.g/l, in particular selected in an interval from about 1 mic.g/ml to about 42 mic.g/ml, more particularly from about 5 mic.g/ml to about 30 mic.g/ml, still more particularly from about 10 mic.g/ml to about 20 mic.g/ml. One example can provide a quantity of the O₂ - O₃ mixture of about 12 mic.g/ml. Another example can provide a quantity of the O₂ - O₃ mixture of about 15 mic.g/ml. Another example can provide a quantity of the O₂ - O₃ mixture of about 18 mic.g/ml.

### EXAMPLE OF THERAPEUTIC TREATMENT OF DEGENERATIVE PATHOLOGY OF THE MYOCARDIUM

Applicant carried out an experiment to show the clinical efficacy of stem cells obtained from de-programming in ozonized whole blood administered in degenerative pathologies of the myocardium.

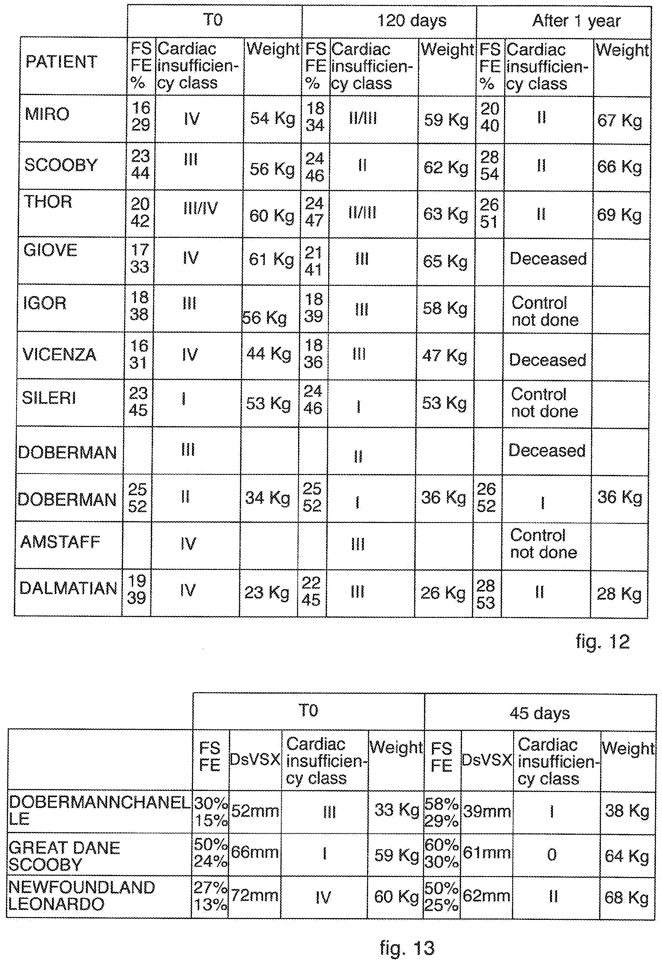

Figs. 12 and 13 are two Tables containing significant parameters of the contractile function to compare the therapeutic results of pathologies with cardiac insufficiency for 11 dogs using stem cells obtained as described in WO-A-2008/034370 and for 3 dogs using stem cells obtained using the method according to the present description, that is, with growth and de-programming using MCSF and ozonization (ozonized whole blood). By comparing the parameters the improvement can clearly be seen in the increase of the contractile capacity using the method described here compared to WO-A-2008/034370, and in particular the fact that this improvement occurs already in the short term (control at 45 days).

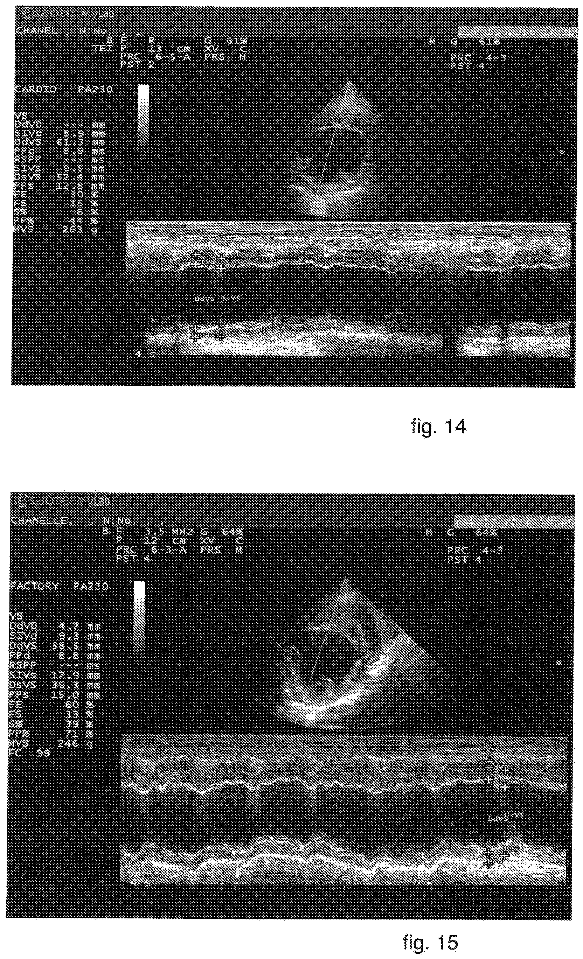

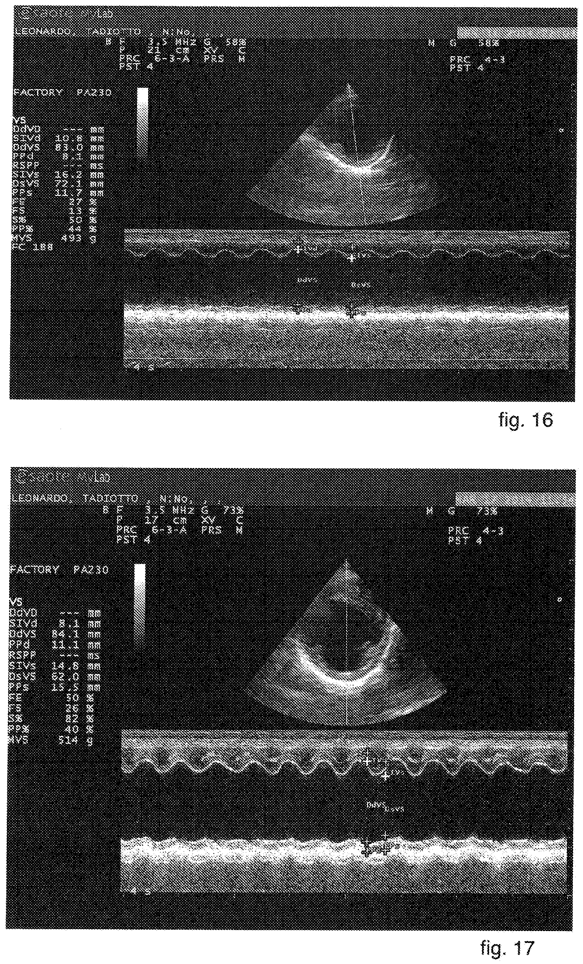

In particular, Applicant treated 3 dogs (1 male Great Dane, 1 male Newfoundland, 1 female Dobermann) of similar ages (6-7) affected by primary dilated myocardiopathy in its advanced stages (a pathology that causes the progressive loss of the contractile function, currently with no possibility of regression using any type of therapy), with severe depression of the contractile function (FS 15-20%). The dogs were treated with expanded stem cells, de-programmed with MCSF, from ozonized whole blood and administered without any purification intravenously and sub-cutaneously in the cardiac zone. The echo-cardiograph images in figs. 14 and 16 show the situation of two different patients (the female Dobermann Chanel and the male Newfoundland Leonardo as per Table in fig. 13) before treatment, where the parameters of contractile function can be seen: extremely negative values of DsVSx (systolic diameter of the left ventricle), FS% and FE%.

On the contrary, as can be seen from the echo-cardiograph images in figs. 15 and 17 for the same two patients, after treatment Applicant found an increase in the contractile capacity measured by linear and volumetric assessment (Teicholz & Simpson method) of the parameters of contractile function DsVSx (systolic diameter of the left ventricle), FS% and FE%. This effect was much more pronounced already in the short term (control at 45 days with respect to the results obtained in the same time period in patients previously treated with the type of purified stem cells obtained from blood as described in WO-A-2008/034370).

Also with regard to the effects caused by the myocardial-degenerative pathology on the general condition and the clinical evaluation of the ISACHS class of heart failure, the improvement found by Applicant was much quicker, with return of appetite, significant weight increase and considerable improvement in the physical performance and resistance to stress already after 1 month.

Applicant therefore concluded that the method for expanding adult stem cells from blood, comprising growth by MCSF and ozonization according to the present invention, allows to recover the contractility of the myocardium that has become deficient due to the degeneration of the same: at the present time, this would have no possibility of recovery with state-of-the-art therapy. In fact, in the veterinary field, other resolutions have been attempted through regenerative medicine, but even inoculating mesenchymal stem cells of different origin (bone marrow, fat) into the myocardium itself has not given significant therapeutic results.

Through stem cells obtained from blood, which have a pluripotent component so that they are able to inter-react with the muscle and its innervation, the results were positive, with a gradual improvement over time. However, the most surprising effect was found with the administration of adult stem cells from whole blood that were ozonized and not purified, intravenously and sub-cutaneously in the cardiac zone, which improved the therapeutic result compared with purified cells, obtaining a better result in a much shorter time.

The echo-cardiograph images in figs. 14, 15, 16 and 17 prove how exceptional the improvements obtained were.

It is clear that modifications and/or additions of parts may be made to the method for expanding adult stem cells from whole blood as described heretofore, without departing from the field and scope of the present invention.

It is also clear that, although the present invention has been described with reference to some specific examples, a person of skill in the art shall certainly be able to achieve many other equivalent forms of method for expanding adult stem cells from whole blood, having the characteristics as set forth in the claims and hence all coming within the field of protection defined thereby.

## Claims

1. Method for expanding adult stem cells from blood, said method comprising:
- growth and de-programming of the adult blood stem cells of a blood sample which has been taken, using in vitro treatment of the blood sample with MCSF;
- ozonization of the blood sample.

2. Method as in claim 1, the method providing that the ozonization of the blood sample is carried out before the MCSF treatment.

3. Method as in claim 1, the method providing that the ozonization of the blood sample is carried out during the MCSF treatment.

4. Method as in claim 1, the method providing that the ozonization of the blood sample is carried out after the MCSF treatment.

5. Method as in any claim hereinbefore, the method providing that the ozonization supplies to the blood sample a mixture of O₂ - O₃.

6. Method as in claim 5, the method providing a stoichiometric ratio of blood to the O₂ - O₃ mixture of 1:1.

7. Method as in claim 5 or 6, the method providing a quantity of O₂ - O₃ mixture in the blood sample greater than or equal to about 1 mic.g/l.

8. Method as in claim 7, the method providing that the quantity of O₂ - O₃ mixture in the blood sample is chosen in an interval from about 1 µg/ml to about 42 µg/ml.

9. Method as in any claim hereinbefore, the method providing to add an anti-coagulant to the blood sample.

10. Method as in any claim hereinbefore, the method providing to use a kit to collect blood which includes a container able to contain the blood taken, containing at least the MCSF substance.

11. Method as in any claim hereinbefore, the method providing that the quantity of blood sample collected and subjected to growth and de-programming with MCSF and ozonization is comprised between 0.2 ml and 100 ml.

12. Method as in any claim hereinbefore, the method providing that the quantity of blood sample collected and subjected to growth and de-programming with MCSF and ozonization is comprised between 2 ml and 10 ml.

13. Method as in any claim hereinbefore, the method providing that the quantity of blood sample collected and subjected to growth and de-programming with MCSF and ozonization is comprised between 3 ml and 5 ml.

14. Method as in any claim hereinbefore, the method providing that the concentration of MCSF is comprised in an interval from about 1 nM to about 55 nM.

15. Method as in any claim hereinbefore, the method providing a growth and de-programming time by means of in vitro treatment with MCSF comprised between 4 hours and 96 hours.

## Patentansprüche

1. Verfahren zum Vermehren von adulten Stammzellen aus Blut, wobei das Verfahren umfasst:
Wachsen und De-Programmieren der adulten Blutstammzellen aus einer entnommenen Blutprobe, wobei eine In-Vitro-Behandlung der Blutprobe mit MCSF angewandt wird; und
Ozonisieren der Blutprobe.

2. Verfahren nach Anspruch 1, wobei das Verfahren vorsieht, dass das Ozonisieren der Blutprobe vor der MCSF-Behandlung durchgeführt wird.

3. Verfahren nach Anspruch 1, wobei das Verfahren vorsieht, dass das Ozonisieren der Blutprobe während der MCSF-Behandlung durchgeführt wird.

4. Verfahren nach Anspruch 1, wobei das Verfahren vorsieht, dass das Ozonisieren der Blutprobe nach der MCSF-Behandlung durchgeführt wird.

5. Verfahren nach einem der vorhergegangenen Ansprüche, wobei das Verfahren vorsieht, dass das Ozonisieren der Blutprobe eine Mischung aus O₂-O₃ zuführt.

6. Verfahren nach Anspruch 5, wobei das Verfahren ein stöchiometrisches Verhältnis von Blut zu der O₂-O₃-Mischung von 1:1 vorsieht.

7. Verfahren nach Anspruch 5 oder 6, wobei das Verfahren eine Menge von O₂-O₃-Mischung in der Blutprobe von größer oder gleich ungefähr 1 mic.g/l vorsieht.

8. Verfahren nach Anspruch 7, wobei das Verfahren vorsieht, dass die Menge von O₂-O₃-Mischung in der Blutprobe aus einer Wertespanne von ungefähr 1 µg/ml bis ungefähr 42 µg/ml gewählt wird.

9. Verfahren nach einem der vorhergegangenen Ansprüche, wobei das Verfahren vorsieht, der Blutprobe einen Gerinnungshemmer zuzugeben.

10. Verfahren nach einem der vorhergegangenen Ansprüche, wobei das Verfahren vorsieht, ein Set zum Sammeln von Blut zu verwenden, welches einen Behälter umfasst, welcher dazu eingerichtet ist, das entnommene Blut zu halten, wobei der Behälter zumindest den MCSF-Wirkstoff enthält.

11. Verfahren nach einem der vorhergegangenen Ansprüche, wobei das Verfahren vorsieht, dass die Menge der gesammelten Blutprobe, welche dem Wachstum und De-Programmieren mit MCSF und Ozonisieren unterzogen wird, zwischen 0,2 ml und 100 ml liegt.

12. Verfahren nach einem der vorhergegangenen Ansprüche, wobei das Verfahren vorsieht, dass die Menge der gesammelten Blutprobe, welche dem Wachstum und De-Programmieren mit MCSF und Ozonisieren unterzogen wird, zwischen 2 ml und 10 ml liegt.

13. Verfahren nach einem der vorhergegangenen Ansprüche, wobei das Verfahren vorsieht, dass die Menge der gesammelten Blutprobe, welche dem Wachstum und De-Programmieren mit MCSF und Ozonisieren unterzogen wird, zwischen 3 ml und 5 ml liegt.

14. Verfahren nach einem der vorhergegangenen Ansprüche, wobei das Verfahren vorsieht, dass die Konzentration von MCSF in einer Wertespanne von ungefähr 1 nM bis ungefähr 55 nM liegt.

15. Verfahren nach einem der vorhergegangenen Ansprüche, wobei das Verfahren eine Wachstums- und De-Programmierungsdauer durch eine In-Vitro-Behandlung mit MCSF zwischen 4 Stunden und 96 Stunden vorsieht.

## Revendications

1. Procédé pour l'expansion de cellules souches adultes à partir de sang, ledit procédé comprenant :
- la croissance et la déprogrammation des cellules souches sanguines adultes d'un échantillon de sang qui a été prélevé, en utilisant un traitement *in vitro* de l'échantillon de sang avec du MCSF ;
- ozonisation de l'échantillon de sang.

2. Procédé selon la revendication 1, le procédé prévoyant que l'ozonisation de l'échantillon sanguin est réalisée avant le traitement par MCSF.

3. Procédé selon la revendication 1, le procédé prévoyant que l'ozonisation de l'échantillon sanguin est réalisée lors du traitement par MCSF.

4. Procédé selon la revendication 1, le procédé prévoyant que l'ozonisation de l'échantillon sanguin est effectuée après le traitement par MCSF.

5. Procédé selon l'une quelconque des revendications précédentes, le procédé prévoyant que l'ozonisation fournit à l'échantillon de sang un mélange d'O₂ - O₃.

6. Procédé selon la revendication 5, le procédé prévoyant un rapport stoechiométrique du sang au mélange O₂- O₃ de 1:1.

7. Procédé selon la revendication 5 ou la revendication 6, le procédé prévoyant une quantité de mélange O₂ - O₃ dans l'échantillon de sang supérieure ou égale à environ 1 mic.g/l.

8. Procédé selon la revendication 7, le procédé prévoyant que la quantité de mélange O₂ - O₃ dans l'échantillon de sang est choisie dans un intervalle d'environ 1 µg/ml à environ 42 µg/ml.

9. Procédé selon l'une quelconque des revendications précédentes, le procédé prévoyant d'ajouter un anticoagulant à l'échantillon de sang.

10. Procédé selon l'une quelconque des revendications précédentes, le procédé prévoyant d'utiliser un kit pour prélever du sang qui comprend un récipient pouvant contenir le sang prélevé, contenant au moins la substance MCSF.

11. Procédé selon l'une quelconque des revendications précédentes, le procédé prévoyant que la quantité d'échantillon de sang recueillie et soumise à une croissance et à une déprogrammation avec du MCSF et une ozonisation est comprise entre 0,2 ml et 100 ml.

12. Procédé selon l'une quelconque des revendications précédentes, le procédé prévoyant que la quantité d'échantillon de sang collectée et soumise à une croissance et à une déprogrammation avec du MCSF et de l'ozonisation est comprise entre 2 ml et 10 ml.

13. Procédé selon l'une quelconque des revendications précédentes, le procédé prévoyant que la quantité d'échantillon de sang recueillie et soumise à une croissance et à une déprogrammation avec du MCSF et de l'ozonisation est comprise entre 3 ml et 5 ml.

14. Procédé selon l'une quelconque des revendications précédentes, le procédé prévoyant que la concentration de MCSF est comprise dans un intervalle allant d'environ 1 nM à environ 55 nM.

15. Procédé selon l'une quelconque des revendications précédentes, le procédé fournissant un temps de croissance et de déprogrammation au moyen d'un traitement *in vitro* avec du MCSF compris entre 4 heures et 96 heures.
